# EUROPEAN PATENT APPLICATION

(11) **EP 2 345 412 A1**
(43) Date of publication of application: **20.07.2011**
(21) Application number: 11001548.4
(22) Date of filing: 04.12.2006
(51) Int. Cl.: A61K 31/675, A61K 38/19, A61P 37/00, A61P 21/04, A61P 25/00

(54) **Use of high-dose oxazaphosphorine drugs for treating immune disorders**

(30) Priority: 02.12.2005 US 742172 P
(62) Divisional of application: 06840105.8
(71) Applicant: The Johns Hopkins University, Baltimore, MD 21218 (US)
(72) Inventor: Brodsky, Robert, A., Brooklandville MD 21022 (US); Jones, Richard, J., Baltimore MD 21230 (US)
(74) Representative: Lock, Graham James

(57) **Abstract**

This disclosure relates, at least in part, to methods of eliminating adverse immune reactions in a subject in need thereof including those associated with autoimmune diseases, allergic reactions and transplant rejection, including administration of a lymphocytotoxic non-myeloablative amount of a oxazaphosphorine drug to the subject.

## Description

### RELATED APPLICATIONS

This application claims the benefit of priority to U.S. Provisional Application No. 60/742,172, filed December 2, 2005.

### BACKGROUND

Autoimmune diseases afflict more than 8 million people in the U.S alone. Autoimmunity usually occurs when the lymphocytes, which are designed to defend the body against infections and foreign agents, start attacking one or more of the body's tissues or organs. Examples of autoimmune diseases include, but are not limited to, systemic lupus erythematosus, rheumatoid arthritis, severe aplastic anemia, multiple sclerosis, autoimmune hemolytic anemia, autoimmune neurologic diseases, type I diabetes, Grave's disease, Crohn's disease, myasthenia gravis, myositis, Raynaud's phenomenon, autoimmune thrombocytopenia, chronic hepatitis and antiphospholipid syndrome.

The conventional treatment for many autoimmune diseases includes the systemic use of anti-inflammatory drugs and potent immunomodulatory agents, such as, for example, steroids, and inhibitors of inflammatory cytokines. However, despite their profound effect on immune responses, these therapies are often unable to induce clinically significant remissions in many patients.

In more recent years, researchers have contemplated the use of stem cells for the treatment of autoimmune diseases, in particular, hematopoietic stem cell transplant therapy (HCST). The rationale is to destroy the mature, long-lived and auto-reactive immune cells and to transplant a new properly functioning immune system into the patient with the hope of eliciting a remission of the autoimmune disease. By it's nature, HSCT is a very risky procedure and for the duration of the recovery phase, until the immune system is reconstituted, transplant recipients undergo a period of dramatically increased susceptibility to bacterial, fungal and viral infections, making this a high-risk therapy. Further, these patients often require extended or life-long immunosuppressive therapy because of reestablishment of the disease caused by the cells that are transplanted and in some instances, onset of graft versus host disease.

High-dose cyclophosphamide (for example, 50 mg/kg/day X 4 days) has also been used for the treatment of certain autoimmune diseases such as, for example, severe aplastic anemia. Severe aplastic anemia (SAA) is a life-threatening bone marrow failure disorder. With supportive care alone, most SAA patients die within a year of diagnosis. Three approaches have generally been used for the treatment of SAA. These are: (1) immunosuppressive therapy; (2) high-dose cyclophosphamide followed by allogeneic bone marrow transplantation; and (3) high-dose cyclophosphamide without bone marrow transplantation.

While low to intermediate doses of cyclophosphamide have been used in an attempt to treat other autoimmune diseases, its use is limited due to the various undesirable side effects. For example, administration of oral daily cyclophosphamide is currently one of the most effective, if not the most effective, immunosuppressive therapy for pemphigus vulgaris. However, the toxicity of cyclophosphamide has limited its use for patients with severe disease who are not responsive to or unable to tolerate nonalkylating agents.

It is unclear whether high-dose cyclophosphamide and similar drugs can be used without any additional therapies for long periods of time and/or whether they can be used for the treatment of all autoimmune and related disorders.

### SUMMARY

This disclosure relates, at least in part, to methods of eliminating or substantially reducing adverse immune reactions or immune disorders in a subject in need thereof including those associated with autoimmune diseases, allergic reactions and transplant rejection, including administration of a lymphocytotoxic non-myeloablative amount of a oxazaphosphorine drug to the subject, such that the subject's immune system reconstitutes without both stem cell transplantation and administration of additional immunomodulatory agents. In some embodiments, the oxazaphosphorine drug is cyclophosphamide.

In some embodiments, a method of treating multiple sclerosis in subject is provided herein which comprises: (a) identifying a subject that failed to respond to conventional therapy; and (b) administering a lymphocytotoxic non-myeloablative amount of a oxazaphosphorine drug to the subject, thereby to treat multiple sclerosis.

In some embodiments, a method oftreating multiple sclerosis comprises: (a) identifying a subject having at least two gadolinium enhancing lesions; and (b) administering a lymphocytotoxic non-myeloablative amount of a oxazaphosphorine drug to the subject, thereby to treat multiple sclerosis.

A method of treating multiple sclerosis described herein is such that the subject's immune system reconstitutes without stem cell transplantation and without administration of additional immunomodulatory agents. In some embodiments, the subject is human. Methods described herein can also be used for treating aggressive relapsing remitting multiple sclerosis.

In some embodiments, a method oftreating multiple sclerosis involves administration of the oxazaphosphorine drug, cyclophosphamide.

In some embodiments, a method of treating an immune disorder excluding severe aplastic anemia, chronic inflammatory demyelinating polyneuropathy, paraneoplastic pemphigus, pemphigus foliaceus, pemphigus vulgaris and/or systemic lupus erthyematosus, includes administering to a subject in need thereof, a lymphocytotoxic non-myeloablative amount of a oxazaphosphorine drug, such that the subject's immune system reconstitutes without stem cell transplantation and such that the disease remains in remission without administration of additional immunomodulatory agents, and where there is no relapse for at least 1 year. In certain embodiments, a method of treating an immune disorder, includes administering to a subject in need thereof, a lymphocytotoxic non-myeloablative amount of a oxazaphosphorine drug, such that the subject's immune system reconstitutes without stem cell transplantation and such that the disease remains in remission without administration of additional immunomodulatory agents, and where there is no relapse for at least 4 years.

In some embodiments, treatment includes curing an immune disorder other than severe aplastic anemia, chronic inflammatory demyelinating polyneuropathy, parancoplastic pemphigus, pemphigus foliaccus, and/or pemphigus vulgaris.

In some embodiments, a method of treating an immune disorder other than severe aplastic anemia further includes the step of administering an effective amount of granulocyte colony stimulating factor to the subject. In certain embodiments, a method of treating an autoimmune disease other than aplastic anemia additionally includes the step of administering an effective amount of at least one antimicrobial agent to the subject. In certain embodiments, a method of treating an autoimmune disease other than aplastic anemia additionally includes the step of administering an effective dose of platelets to the subject. A method of treating an autoimmune disease, as described herein, may include any one, two or all three of these additional steps.

In some embodiments, an effective amount of platelets are administered to a subject for a duration of time necessary for the platelet count to be at least 10,000 platelets/mm³ and an effective amount of granulocyte colony stimulating factor is administered for a duration of time necessary for the neutrophil count to be at least 500/mm³. In some embodiments, an effective amount of red blood cells are administered to a subject for a duration of time necessary for the hemoglobin to be maintained at least at 8.0 g/dI.

In some embodiments, a method encompassed by this disclosure includes a method for treating a subject having an immune disorder other than paraneoplastic pemphigus, pemphigus foliaceus, or pemphigus vulgaris, including administering a lymphocytotoxic non-myeloablative amount of a oxazaphosphorine drug to the subject, such that the subject's immune system reconstitutes without both stem cell transplantation and administration of additional immunomodulatory agents, and where the method does not include administration of platelets.

In some methods encompassed by this disclosure, an effective amount of granulocyte colony stimulating factor is 5 µg/kg/day, which is administered for a duration of time necessary for the neutrophil count to be at least 1000/mm³. In some embodiments, methods encompassed by this disclosure include administration of an effective amount of NEULASTA®.

In some embodiments, a method of treating an immune disorder includes administering to a subject in need thereof, a lymphocytotoxic non-myeloablative amount of a oxazaphosphorine drug followed by, administering an effective amount of granulocyte colony stimulating factor to the subject; and administering an effective amount of at least one antimicrobial agent to the subject, where the method does not include all three of (a) stem cell transplantation; (b) administration of additional immunomodulatory agents; and (c) administration of platelets.

In further embodiments, this disclosure relates to a method of obtaining a cell population substantially free of cells capable of eliciting an adverse immune reaction in a subject, including: (a) administering a lymphocytotoxic non-myeloablative amount of a oxazaphosphorinc drug to the subject, followed by, (b) administering an effective amount of granulocyte colony stimulating factor to the subject; (c) administering an effective amount of at least one antimicrobial agent to the subject; and (d) administering an effective amount of platelets to the subject, where the method does not include the use of both stem cell transplantation and administration of additional immunomodulatory agents. Exemplary additional immunomodulatory agents include but are not limited to, for example, prednisone, cyclosporine, methotrexate, tacrolimus, pimecrolimus and azathioprine. The high dose cyclophosphamide therapy described herein is more effective than the low-dose therapy, which usually requires daily oral dosing or monthly intravenous pulses at 500-1000 mg/m² and has a higher risk of malignancies and premature menopause and/or infertility.

In addition to autoimmune diseases, this disclosure also encompasses the treatment of other adverse immune reactions such as allergic reactions and transplant rejections. Examples of allergic reactions which may be treated using methods described herein include, but are not limited to, for example, systemic allergic reactions, allergic reactions to immunotherapy, anaphylactic reactions, atopic disease, contrast allergy, drug allergies, food allergies such as, for example, shellfish and peanut allergies, hypersensitivity reactions, insect sting allergies, latex allergy, penicillin allergy, and radiocontrast medium allergy.

Examples of transplant rejections which may be treated using methods described herein include, for example, transplant rejection occurring during or following allogenic antigen transplantation of organs, tissues, or cells into a host; transplant rejection occurring during or following a xenogenic transplantation of organs, tissues, or cells into a host; and transplant rejection occurring during or following transplantation of autologous tissue, organs or cells into a host. Transplant rejections also include rejections occurring during or following transplantation of an organ, tissue or hematopoietic stem cells from related (matched or partially matched) or unrelated donors. Transplant rejections after stem cell transplantation include both graft rejection and graft-versus-host disease without wishing to be bound by theory, it is contemplated that any disease which can be effectively treated by eliminating the subject's circulating immune cells with high dose cyclophosphamide and allowing them to redevelop from hematopoietic stem cells is encompassed by this disclosure.

Accordingly, diseases which may be treated by the methods described herein include, but are not limited to, AIDS-associated myopathy, AIDS-associated neuropathy, Acute disseminated encephalomyelitis, Addison's Disease, Alopecia Areata, Anaphylaxis Reactions, Ankylosing Spondylitis, Antibody-related Neuropathies, Antiphospholipid Syndrome, Autism, Autoimmune Atherosclerosis, Autoimmune Diabetes Insipidus, Autoimmune Endometriosis, Autoimmune Eye Diseases, Autoimmune Gastritis, Autoimmune Hemolytic Anemia, Autoimmune Hemophilia, Autoimmune Hepatitis, Autoimmune Interstitial Cystitis, Autoimmune Lymphoproliferative Syndrome, Autoimmune Myelopathy, Autoimmune Myocarditis, Autoimmune Neuropathies, Autoimmune Oophoritis, Autoimmune Orchitis, Autoimmune Thombocytopenia, Autoimmune Thyroid Diseases, Autoimmune Urticaria, Autoimmune Uveitis, Autoimmune Vasculitis, Behcet's Disease, Bell's Palsy, Bullous Pemphigoid, CREST, Celiac Disease, Ccrcbcllar degeneration (parancoplastic), Chronic Fatigue Syndrome, Chronic Rhinosinusitis, Chronic inflammatory demyelinating polyneuropathy, Churg Strauss Syndrome, Connective Tissue Diseases, Crohn's Disease, Cutaneous Lupus, Dermatitis Herpetiformis, Dermatomyositis, Diabetes Mellitus, Discoid Lupus Erythematosus, Drug-induced Lupus, Endocrine Orbitopathy, Glomerulonephritis, Goodpasture Syndrome, Goodpasture's Syndrome, Graves Disease, Guillian-Barre Syndrome, Miller Fisher variant of the Guillian Barre Syndrome, axonal Guillian Barre Syndrome, demyelinating Guillian Barre Syndrome, Hashimoto Thyroiditis, Herpes Gestationis, Human T-cell lymphomavims-associated myelopathy, Huntington's Disease, IgA Nephropathy, Immune Thrombocytopenic Purpura, Inclusion body myositis, Interstitial Cystitis, Isaacs syndrome, Lambert Eaton myasthenic syndrome, Limbic encephalitis, Lower motor neuron disease, Lyme Disease, MCTD, Microscopic Polyangiitis, Miller Fisher Syndrome, Mixed Connective Tissue Disease, Mononeuritis multiplex (vasculitis), Multiple Sclerosis, Myasthenia Gravis, Myxedema, Meniere Disease, Neonatal LE, Neuropathies with dysproteinemias, Opsoclonus-myoclonus, PBC, POEMS syndrome, Paraneoplastic Autoimmune Syndromes, Pemphigus, Pemphigus Foliaceus, Pemphigus Vulgaris, Pernicious Anemia, Peyronie's Disease, Plasmacytoma/myeloma neuropathy, Poly-Dermatomyositis, Polyarteritis Nodosa, Polyendocrine Deficiency Syndrome, Polyendocrine Deficiency Syndrome Type 1, Polyendocrine Deficiency Syndrome Type 2, Polyglandular Autoimmune Syndrome Type I, Polyglandular Autoimmune Syndrome Type II, Polyglandular Autoimmune Syndrome Type III, Polymyositis, Primary Biliary Cirrhosis, Primary Glomerulonephritis, Primary Sclerosing Cholangitis, Psoriasis, Psoriatic Arthritis, Rasmussen's Encephalitis, Raynaud's Discase, Relapsing Polychondritis, Retrobulbar neuritis, Rheumatic Diseases, Rheumatoid Arthritis, Scleroderma, Sensory neuropathies (paraneoplastic), Sjogren's Syndrome, Stiff-Person Syndrome, Subacute Thyroiditis, Subacute autonomic neuropathy, Sydenham Chorea, Sympathetic Ophthalmitis, Systemic Lupus Erythematosus, Transverse myelitis, Type 1 Diabetes, Ulcerative Colitis, Vasculitis, Vitiligo, Wegener's Granulomatosis, Acrocyanosis, Anaphylactic reaction, Autoimmune inner ear disease, Bilateral sensorineural hearing loss, Cold agglutinin hemolytic anemia, Cold-induced immune hemolytic anemia, Idiopathic endolymphatic hydrops, Idiopathic progressive bilateral sensorineural hearing loss, Immune-mediated inner ear disease, and Mixed autoimmune hemolysis.

In some embodiments, this disclosure relates to the treatment of scleroderma in a subject including administration of a lymphocytotoxic non-mycloablativc amount of a oxazaphosphorine drug to the subject, thereby to treat scleroderma.

In some embodiments, a lymphocytotoxic non-myeloablative amount of a oxazaphosphorine drug used in the methods described herein is between 100 mg/kg and 200 mg/kg, administered daily from 1 to 7 days. In some embodiments, a lymphocytotoxic non-myeloablative amount of a oxazaphosphorine drug is between 25 mg/kg and 100 mg/kg, administered daily for 4 consecutive days. In certain embodiments, a lymphocytotoxic non-myeloablative amount of a oxazaphosphorine drug is 50 mg/kg administered daily for 4 consecutive days.

Exemplary oxazaphosphorine drugs include, but are not limited to, cyclophosphamide, ifosfamide, perfosfamide, trophosphamide (trofosfamide), or a pharmaceutically acceptable salt, solvate, prodrug and metabolite thereof. In some embodiments, a oxazaphosphorine drug used in the methods described herein is cyclophosphamide or a pharmaceutically acceptable salt or metabolite thereof.

Exemplary antimicrobial drugs used in the methods described herein include, but are not limited to, Amdinocillin (Mecillinam), Amikacin, Amoxicillin, Ampicillin, Azithromycin, Aztreonam, Bacampicillin, Bacitracin, Carbenicillin indanyl sodium, Cefaclor, Cefadroxil, Cefamandole, Cefazolin, Cefdinir, Cefditoren, Cefepime, Cefixime, Cefmetazole, Cefonicid, Cefoperazone, Cefotaxime, Cefotetan, Cefoxitin, Cefpodoxime Proxetil, Cefprozil, Ceftazidime, Ceftibuten, Ceftizoxime, Ceftriaxone, Cefuroxime, Cefuroxime axetil, Cephalexin, Cephalothin, Cephapirin, Cephradine, Chloramphenicol, Cnnoxacin, Ciprofloxacin, Clarithromycin, Clindamycin, Cloxacillin, Colistimethate, Daptomycin, Demeclocycline, Dicloxacillin, Dirithromycin, Doxycycline, Enoxacin, Ertapenem, Erythromycin, Fosfomycin, Gatifloxacin, Gemifloxacin, Gentamicin, Grepafloxacin, Imipenem/Cilastatin, Kanamycin, Levofloxacin, Lincomycin, Linezolid, Lomefloxacin, Loracarbef, Mafenide, Meropenem, Methacycline, Methenamine mandelate, Methenamine hippurate, Methicillin, Metronidazole, Mezlocillin, Minocycline, Moxifloxacin, Mupirocin, Nafcillin, Nalidixic Acid, Neomycin, Netilmycin, Nitrofurantoin, Nitrofurazone, Norfloxacin, Novobiocin, Ofloxacin, Oxacillin, Oxytetracycline, Penicillin, Piperacillin, Polymyxin B, Rifamixin, Sparfloxacin, Spectinomycin, Streptomycin, Sulfadiazine, Sulfamethoxazole, Sulfisoxazole, Teicoplanin, Telithromycin, Tetracycline, Ticarcillin, Tobramycin, Trimethoprim, Trovafloxacin, Vancomycin, and a pharmaceutically acceptable salt or derivative thereof.

Exemplary combinations of antimicrobial agents include, but are not limited to, for example, Amoxicillin plus Clavulanate, Ticarcillin plus Clavulanic Acid, Trimethoprim plus Sulfamethoxazole, Piperacillin plus Tazobactam, Quinupristin plus Dalfopristin, and Ampicillin plus Sulbactam.

In certain embodiments, an antimicrobial agent is chosen from the group consisting of Amphotericin B, Amphotericin B Deoxycholate, Amphotericin B cholesteryl sulfate complex (ABCD), Amphotericin B lipid complex (ABLC), Amphotericin B liposomal, Caspofungin acetate, Clotrimazole, Fluconazole, Flucytosine, Griseofulvin, Itraconazole, Ketoconazole, Miconazole, Nystatin, Pentamidine, Terbinafine, and Voriconazole.

In some embodiments, methods encompassed by this disclosure further include administration of an antiviral drug. Antiviral drugs include, but are not limited to, Abacavir, Aciclovir, Amantadine, Didanosine, Emtricitabine, Enfuvirtide, Entecavir, Lamivudine, Nevirapine, Ribavirin, Rimantidine, Stavudine, Valaciclovir, Vidarabine, Zalcitabine, and Zidovudine.

Also encompassed by this disclosure is a kit for treating an immune disorder including: (a) a plurality of doses of a non-myeloablative oxazaphosphorine drug; and (b) instructions for treating the immune disorder using one or more doses of the oxazaphosphorine drug; wherein the one or more doses are lymphocytotoxic.

In some embodiments, a kit for treating an immune disorder further includes one or more of: (a) a plurality of doses of granulocyte colony stimulating factor; (b) a plurality of doses of platelets; and (d) a plurality of doses of one or more antimicrobial agent.

The kits encompassed by this disclosure can be used for treating an immune disorder chosen from an autoimmune disease, an allergic reaction and transplant rejection.

### DETAILED DESCRIPTION

This disclosure is based, at least in part, on the discovery that administration of a lymphocytotoxic non-myeloablative amount of a oxazaphosphorine drug can be used for replacing a subject's immune cells, including autoreactive lymphocytes, with disease-free immune cells, without the use of both stem cell transplantation and additional immunomodulatory agents.

The rationale underlying this approach is the discovery that oxazaphosphorine drugs such as cyclophosphamide are lymphocytotoxic but spare hematopoietic progenitor stem cells because of high levels of aldehyde dehydrogenase, an enzyme, which confers resistance to cyclophosphamide.

High-dose cyclophosphamide was originally used in allogeneic bone marrow transplantation because of its ability to break immune tolerance and facilitate engraftment. (See, for example, Santos et al., Transplant Proc., 4: 559-564 (1972)).

As a prodrug, cyclophosphamide is converted to 4-hydroxycyclophosphamide and its tautomer aldophosphamide in the liver. These compounds diffuse into cells and are converted into the active compound phosphoramide mustard. Alternatively, they are inactivated by the enzyme aldehyde dehydrogenase to form the inert carboxyphosphamide. Lymphoid cells, including NK cells, and B and T lymphocytes, have low levels of aldehyde dehydrogenase and are rapidly killed by high doses (i.e., lymphocytotoxic) of cyclophosphamide. In contrast, hematopoietic progenitor stem cells possess high levels of aldehyde dehydrogenase, rendering them resistant to cyclophophamide. (See, for example, Hilton, Cancer Res., 44:5156-60 (1984); Kastan et al., Blood, 73:1947-50 (1990); Zoumbos et al., N. Eng. J. Med., 312:257-265 (1985); Brodsky, Sci. World J., 2: 1808-15 (2002)).

### 1. DEFINITIONS

In order that the present disclosure may be more readily understood, certain terms are first defined. Additional definitions are set forth throughout the detailed description.

The term "relapse" refers to the recurrence of an immune disorder after recovery following treatment; and or recurrence of one or more symptoms associated with an immune disorder after recovery following treatment. No relapse for at least about four years is intended to include no relapse between about 3.5 years to about 4.5 years. No relapse for at least about five years is intended to include no relapse between about 4.5 to about 5.5 years. No relapse for at least about ten years is intended to include no relapse between about 9 to about 11 years.

The term "remission" refers to the disappearance of autorcactivc cells following treatment and/or disappearance of one or more or all symptoms associated with an adverse immune reaction, including, for example, an autoimmune disease, an allergic reaction and transplant rejection.

As used herein, the phrase "a lymphocytotoxic non-myeloablative amount of a oxazaphosphorine drug" refers to an amount of the drug which is immunoablative, upon single or multiple dose administration to a subject (such as a human patient suffering from an autoimmune disease, an allergic reaction or transplant rejection), thereby resulting in a substantial reduction in or complete elimination of mature circulating lymphocytes in the subject. In some embodiments, administration of a lymphocytotoxic non-myeloablative amount of a oxazaphosphorine drug results in treating, preventing, curing, delaying, reducing the severity of, ameliorating at least one symptom of a disorder or recurring disorder, or prolonging the survival of the subject beyond that expected in the absence of such administration. In some embodiments, "a lymphocytotoxic non-myeloablative amount of an oxazaphosphorine drug" refers to a dose of the drug administered to a subject in need thereof, which results in eliminating or substantially reducing the number of circulating lymphocytes in the subject, including those which are associated with an adverse immune reaction such as, for example, an autoimmune disease, transplant rejection and allergic reaction, while sparing the hematopoietic progenitor stem cells. For example, in some embodiments, "a lymphocytotoxic non-myeloablative amount of a oxazaphosphorine drug" is a 50 mg/kg/day dose of cyclophosphamide administered to a subject in need thereof for 4 consecutive days.

The phrase "granulocyte colony stimulating factor" or "GCSF" refers to a hematopoietic growth factor that stimulates the development of committed progenitor cells to neutrophils and enhances the functional activities of neutrophils. It is produced in response to specific stimulation by a variety of cells including macrophages, fibroblasts, endothelial cells and bone marrow stroma. GCSF can be used clinically to facilitate hematopoietic recovery after bone marrow transplantation. In some embodiments described herein, GCSF is administered to increase the neutrophil count to a level, which falls within a normal range. Either purified GCSF or recombinant GCSF, for example, recombinant human GCSF (R & D SYSTEMS, INC, Minneapolis, MN) can be used in the methods described herein.

The phrase "an effective amount of granulocyte colony stimulating factor" refers to an amount of granulocyte colony stimulating factor, which upon a single or multiple dose administration to a subject, results in an elevation in neutrophil count in the subject. Elevation in neutrophil count includes any measurable increase in neutrophil count or appearance of neutrophils following administration of an effective amount of granulocyte colony stimulating factor. A measurable increase can be, for example, a 5-fold, or a 10-fold, or a 15-fold, or a 20-fold, or a 25-fold, or a 30-fold, or a 40-fold, or a 50-fold, or a 60-fold, or a 70-fold, or a 80-fold, or a 90-fold, or a 100-fold, or greater than a 100-fold increase in neutrophil count following administration of an effective amount of granulocyte colony stimulating factor. In some embodiments, an elevation in neutrophil count includes elevation to a count that is within a normal range, as understood by one of ordinary skill in the art. In some embodiments, "an effective amount of granulocyte colony stimulating factor" refers to a daily administration of 5 µg/kg of the granulocyte colony stimulating factor.

The phrase "an effective amount of an antimicrobial agent" refers to an amount of one or more than one agent with an antimicrobial activity, which upon a single or multiple dose administration to a subject, results in an elevation in leukocyte count in the subject. Elevation in leukocyte count includes any measurable increase in leukocyte count or appearance of leukocytes following administration of an effective amount of an antimicrobial agent. A measurable increase can be, for example, a 5-fold, or a 10-fold, or a 15-fold, or a 20-fold, or a 25-fold, or a 30-fold, or a 40-fold, or a 50-fold, or a 60-fold, or a 70-fold, or a 80-fold, or a 90-fold, or a 100-fold, or greater than a 100-fold increase in leukocyte count following administration of an effective amount of at least one antimicrobial agent. In some embodiments, an elevation in leukocyte count includes elevation to a count which is within a normal range, as understood by one or ordinary skill in the art.

The term "within a normal range" refers to a certain measurement, for example, number of cells or cell count, in a healthy subject. It would be apparent to one of ordinary skill in the art whether a particular indicia being measured is within "a normal range."

The term "use of additional immunomodulatory agents," as used herein, refers to the use of any agent, other than a oxazaphosphorine drug, which is capable of modulating the immune system (e.g., by increasing or decreasing an immune response; increasing or decreasing activity of one or more immune cells and/or activating or suppressing the immune system), in the methods described herein. For example, in some embodiments, immunomodulatory agents include immunosuppressive agents, other than a oxazaphosphorine agent such as cyclophosphamide, which when administered at an appropriate dosage, results in the inhibition of an immune response, for example, inhibition of T cell activity. Examples of such agents include, but are not limited to, prednisone, cyclosporine, FK-506, and rapamycin. In some embodiments, exclusion of any additional immunomodulatory agents in methods described herein, refers to exclusion of additional immunosuppressive agents subsequent to, or concurrently with the administration of a lymphocytotoxic non-myeloablative amount of a oxazaphosphorine drug.

Methods which do not include the use of "any additional immunomodulatory agents," specifically exclude the use of agents which are immunosuppressive, such as, for example, prednisone, in methods which use a lymphocytotoxic non-myeloablative amount of a oxazaphosphorine drug.

The terms "treat," "treating," and "treatment," as used herein, refer to therapeutic or preventative measures described herein. The methods of "treatment" employ administration to a subject in need thereof such as, for example, a subject having an autoimmune disease, an allergic reaction or transplant rejection, or who ultimately may acquire a disorder such as, for example, an autoimmune disease, an allergic reaction or transplant rejection, a lymphocytotoxic non-myeloablative amount of a oxazaphosphorine drug, such as, for example, cyclophosphamide, in order to prevent, cure, delay, reduce the severity of, or ameliorate one or more symptoms of the disorder or recurring disorder, or in order to prolong the survival of a subject beyond that expected in the absence of such treatment.

The terms "cure" and "curing," as used herein, refer to a remission of a disease or an elimination of symptoms (e.g., clinical, laboratory, and imaging) of a disease in a subject such as, for example, an autoimmune disease, an allergic reaction or transplant rejection, by the methods described herein. The remission of a disease or the elimination of symptoms of a disease in a subject maybe for at least about 1 year, at least about 2 years, at least about 3 years, at least about 4 years, or at least about 5 years. In certain embodiments, a remission of a disease or an elimination of symptoms of a disease in a subject includes the absence of administering alternative methods of treatment such as immunosuppressants (e.g., cyclosporinc, cyclophosphamide, etc), and/or steroids. In some embodiments, a method of curing an immune disorder includes administration of a lymphocytotoxic non-myeloablative amount of an oxazaphosphorine drug to a subject in need thereof, where the immune disorder is not severe aplastic anemia, chronic inflammatory demyelinating polyneuropathy, paraneoplastic pemphigus, paraneoplastic pemphigus, pemphigus foliaceus, or pemphigus vulgaris.

The term "an oxazaphosphorine drug" refers to a class of drugs, which act as alkylating agents and cause immunoablation. They are generally highly cytotoxic and are often used as chemotherapeutic agents. Examples of oxazaphosphorine drugs include cyclophosphamide, ifosfamide, perfosfamide, trophosphamide (trofosfamide), and pharmaceutically acceptable salts, solvates, prodrugs and metabolites thereof. In some embodiments, an oxazaphosphorine drug used in the methods described herein is cyclophosphamide, which is sold under common trade-names including PROCYTOX®, CYTOXAN® and NEOSAR®. As discussed above, cyclophosphamide is converted to 4-hydroxycyclophosphamide and its tautomer aldophosphamide in the liver and is cytotoxic to cells that express low levels of the enzyme aldehyde dehydrogenase, for example, NK cells and T and B lymphocytes.

Ifosfamide (MITOXANAO) is a structural analog of cyclophosphamide and its mechanism of action is considered to be identical or substantially similar to that of cyclophosphamide. Perfosfamide (4hydroperoxycyclophosphamide) and trophosphamide are also alkylating agents, which are structurally related to cyclophosphamide. For example, perfosfamide alkylates DNA, thereby inhibiting DNA replication and RNA and protein synthesis.

The term "hematopoietic progenitor stem cell," as used herein refers to any type of cell of the hematopoietic system, including, but not limited to, undifferentiated cells such as hematopoietic stem cells and progenitor cells, which are capable ofreconstituting the immune system following administration of a lymphocytotoxic non-myeloablative amount of a oxazaphosphorine drug, as described herein.

The terms "immunoablation" and "immunoablative," as used herein, refer to severe immunosuppression using a high-dose (i.e., lymphocytotoxic non-myeloablative amount) of a oxazaphosphorine drug such as, for example, 50 mg/kg X 4 days of cyclophosphamide, which leads to substantial reduction in or elimination of the population of circulating lymphocytes, including for example, NK cells and B and T lymphocytes. Immunoablation, as described herein, results in complete or substantially complete reduction in autoreactive antibodies and memory cells responsible for an autoimmune response.

The term "lymphocytotoxic," as used herein, refers to complete elimination of or substantial reduction in the number of circulating lymphocytes, including those associated with an adverse immune reaction in a subject, such as, for example, an autoimmune disease, an allergic reaction and transplant rejection in a subject following administration of a high-dose (i.e., lymphocytotoxic non-mycloablative amount) of a oxazaphosphorine drug, such as, for example, 50 mg/kg X 4 days of cyclophosphamide. Substantial reduction can be a reduction of about 5%, 10%, 15%, 20%, 25%, 50%, 75%, 90%, 95%, 98%, 99% of the circulating lymphocytes. The term "lymphocytotoxic," includes killing of those immune cells by a oxazaphosphorine drug which express low levels of the enzyme aldehyde dehydrogenase.

The term "non-myeloablative," as used herein, refers to a property of a compound such as, for example, an oxazaphosphorine drug such as cyclophosphamide, whereby the compound does not have a detectable or significant cytotoxic effect on myeloid cells, for example, hematopoietic progenitor stem cells. In some embodiments, a non-myeloablative agent used in the methods described herein has a cytotoxic effect on the circulating mature lymphocytes (e.g., NK cells, and T and B lymphocytes) while sparing the progenitor cells, e.g., hematopoietic progenitor stem cells that are capable of reconstituting the immune system. In some embodiments, a non-myeloablative agent used in the methods of the invention kills cells which express low levels of the enzyme aldehyde dehydrogenase (e.g., NK cells and B and T lymphocytes) while sparing cells which express high levels of the enzyme aldehyde dehydrogenase (e.g., hematopoietic progenitor stem cells).

### II. EXEMPLARY DISORDERS

Various methods described herein can be used for treating autoimmune diseases, allergic reactions and transplant rejection.

Exemplary autoimmune diseases which can be treated using methods described herein include, but are not limited to, AIDS-associated myopathy, AIDS-associated neuropathy, Acute disseminated encephalomyelitis, Addison's Disease, Alopecia Areata, Anaphylaxis Reactions, Ankylosing Spondylitis, Antibody-related Neuropathies, Antiphospholipid Syndrome, Autism, Autoimmune Atherosclerosis, Autoimmune Diabetes Insipidus, Autoimmune Endometriosis, Autoimmune Eye Diseases, Autoimmune Gastritis, Autoimmune Hemolytic Anemia, Autoimmune Hemophilia, Autoimmune Hepatitis, Autoimmune Interstitial Cystitis, Autoimmune Lymphoproliferative Syndrome, Autoimmune Myelopathy, Autoimmune Myocarditis, Autoimmune Neuropathies, Autoimmune Oophoritis, Autoimmune Orchitis, Autoimmune Thrombocytopenia, Autoimmune Thyroid Diseases, Autoimmune Urticaria, Autoimmune Uveitis, Autoimmune Vasculitis, Behcet's Disease, Bell's Palsy, Bullous Pemphigoid, CREST, Celiac Disease, Cerebellar degeneration (paraneoplastic), Chronic Fatigue Syndrome, Chronic Rhinosinusitis, Chronic inflammatory demyelinating polyneuropathy, Churg Strauss Syndrome, Connective Tissue Diseases, Crohn's Disease, Cutaneous Lupus, Dermatitis Herpetiformis, Dermatomyositis, Diabetes Mellitus, Discoid Lupus Erythematosus, Druginduced Lupus, Endocrine Orbitopathy, Glomerulonephritis, Goodpasture Syndrome, Goodpasture's Syndrome, Graves Disease, Guillain-Barre Syndrome, Guillian Barre Syndrome (Miller Fisher variant), Guillian Barre Syndrome (axonal), Guillian Barre Syndrome (demyelinating), Hashimoto's Thyroiditis, Herpes Gestationis, Human T-cell lymphomavirus-associated myelopathy, Huntington's Disease, IgA Nephropathy, Immune Thrombocytopenic Purpura, Inclusion body myositis, Interstitial Cystitis, Isaacs syndrome, Lambert Eaton myasthenic syndrome, Limbic encephalitis, Lower motor neuron disease, Lyme Disease, MCTD, Microscopic Polyangiitis, Miller Fisher Syndrome, Mixed Connective Tissue Disease, Mononcuritis multiplex (vasculitis), Multiple Sclerosis, Myasthenia Gravis, Myxedema, Meniere Disease, Neonatal LE, Neuropathies with dysproteinemias, Opsoclonus-myoclonus, PBC, POEMS syndrome, Paraneoplastic Autoimmune Syndromes, Pemphigus, Pemphigus Foliaceus, Pemphigus Vulgaris, Pernicious Anemia, Peyronie's Disease, Plasmacytoma/myeloma neuropathy, Poly-Dermatomyositis, Polyarteritis Nodosa, Polyendocrine Deficiency Syndrome, Polyendocrine Deficiency Syndrome Type 1, Polyendocrine Deficiency Syndrome Type 2, Polyglandular AutoimmuneSyndrome Type I, Polyglandular Autoimmune Syndrome Type T1, Polyglandular Autoimmune Syndrome Type III, Polymyositis, Primary Biliary Cirrhosis, Primary Glomerulonephritis, Primary Sclerosing Cholangitis, Psoriasis, Psoriatic Arthritis, Rasmussen's Encephalitis, Raynaud's Disease, Relapsing Polychondritis, Retrobulbar neuritis, Rheumatic Diseases, Rheumatoid Arthritis, Scleroderma, Sensory neuropathies (para neo plastic), Sjogren's Syndrome, Stiff-Person Syndrome, Subacute Thyroiditis, Subacute autonomic neuropathy, Sydenham Chorea, Sympathetic Ophthalmitis, Systemic Lupus Erythematosus, Transverse myelitis, Type 1 Diabetes, Ulcerative Colitis, Vasculitis, Vitiligo, Wegener's Granulomatosis, Acrocyanosis, Anaphylactic reaction, Autoimmune inner ear disease, Bilateral sensorineural hearing loss, Cold agglutinin hemolytic anemia, Cold-induced immune hemolytic anemia, Idiopathic endolymphatic hydrops, Idiopathic progressive bilateral sensorineural hearing loss, Immune-mediated inner ear disease, and Mixed autoimmune hemolysis.

Without wishing to be bound by theory, it is understood that methods described herein can be used for treating any immune disorder in which it would be desirable to replace the circulating auto-reactive lymphocytes with disease free immune cells. One of ordinary skill in the art can easily determine which diseases fall in this category, for example, by detecting auto-reactive antibodies or antibodies which react with self-antigens in a subject suffering from such a disease. Alternatively, by detecting cells in a subject which are capable of mounting an immune response against a self-antigen in the subject. Methods of diagnosing one or more autoimmune diseases encompassed by this disclosure are well-known in the art and can easily be performed by a skilled artisan.

In addition to autoimmune diseases, also encompassed by this disclosure are allergic reactions, which can be treated by methods described herein. Exemplary allergic reactions include, but are not limited to, systemic allergic reaction, an allergic reaction to immunotherapy, anaphylactic reaction, atopic disease, contrast allergy, drug allergy, food allergy, hypersensitivity reaction, insect sting allergy, latex allergy, penicillin allergy, and radiocontrast medium allergy. Examples of food allergies include an allergic reaction to peanuts or shellfish, for example.

In addition to autoimmune diseases and allergic reactions, also encompassed by this disclosure are transplant rejections that can be treated using methods described herein. For example, in some embodiments, transplant rejection occurring during or following an allogenic antigen transplantation of organs, tissues, or cells into a host can be treated using methods described herein. In certain embodiments, transplant rejection occurring during or following a xenogenic transplantation of organs, tissues, or cells into a host can be treated using methods described herein. In certain embodiments, transplant rejection occurring during or following transplantation of autologous tissue, organs or cells into a host can be treated using methods described herein.

Also encompassed by this disclosure are transplant rejections occurring during or following a transplant of an organ, tissue or cells from a half-matched donor, which usually results in graft versus host disease.

### III. EXEMPLARY ANTIMICROBIAL AND ANTIVIRAL AGENTS

Exemplary antimicrobial drugs used in the methods described herein include, but are not limited to, Amdinocillin (Mecillinam), Amikacin, Amoxicillin, Ampicillin, Azithromycin, Aztreonam, Bacampicillin, Bacitracin, Carbenicillin indanyl sodium, Cefaclor, Cefadroxil, Cefamandole, Cefazolin, Cefdinir, Cefditoren, Cefepime, Cefixime, Cefmetazole, Cefonicid, Cefoperazone, Cefotaxime, Cefotetan, Cefoxitin, Cefpodoxime Proxetil, Cefprozil, Ceftazidime, Ceftibuten, Ceftizoxime, Ceftriaxone, Cefuroxime, Cefuroxime axetil, Cephalexin, Cephalothin, Cephapirin, Cephradine, Chloramphenicol, Cinoxacin, Ciprofloxacin, Clarithromycin, Clindamycin, Cloxacillin, Colistimethate, Daptomycin, Demeclocycline, Dicloxacillin, Dirithromycin, Doxycycline, Enoxacin, Ertapenem, Erythromycin, Fosfomycin, Gatifloxacin, Gemifloxacin, Gentamicin, Grepafloxacin, Imipenem/Cilastatin, Kanamycin, Levofloxacin, Lincomycin, Linezolid, Lomefloxacin, Loracarbef, Mafenide, Meropenem, Methacycline, Methenamine mandelate, Methenaminehippurate, Methicillin, Metronidazole, Mezlocillin, Minocycline, Moxifloxacin, Mupirocin, Nafcillin, Nalidixic Acid, Neomycin, Netilmycin, Nitrofurantoin, Nitrofurazone, Norfloxacin, Novobiocin, Ofloxacin, Oxacillin, Oxytetracycline, Penicillin, Piperacillin, Polymyxin B, Rifamixin, Sparfloxacin, Spectinomycin, Streptomycin, Sulfadiazine, Sulfamethoxazole, Sulfisoxazole, Teicoplanin, Telithromycin, Tetracycline, Ticarcillin, Tobramycin, Trimethoprim, Trovafloxacin, Vancomycin and a pharmaceutically acceptable salt or derivative thereof

Various anti-microbial agents used in the methods described herein can either be used alone or in combination with another antimicrobial agent, so long as the antimicrobial agents alone or in combination result in an increase in leukocyte count which is within a normal range and so long as the antimicrobial agents do not have an adverse reaction with each other or with any other compounds administered in the methods described herein. One skilled in the art can easily determine whether to use a single antimicrobial agent in the methods or a combination of agents using the standard techniques known in the art and those described herein. In some embodiments, choice of an antimicrobial agent may depend on the susceptibility of a subject being treated to an infection, for example, a bacterial infection. In certain embodiments, choice of an antimicrobial agent may depend on the occurrence of such an infection in the subject being treated.

Exemplary combinations of antimicrobial agents include, but are not limited to, for example, Amoxicillin plus Clavulanate, Ticarcillin plus Clavulanic Acid, Trimethoprim plus Sulfamethoxazole, Piperacillin plus Tazobactam, Quinupristin plus Dalfopristin, and Ampicillin plus Sulbactam.

In certain embodiments, an antimicrobial agent is chosen from the group consisting of Amphotericin B, Amphotericin B Deoxycholate, Amphotericin B cholesteryl sulfate complex (ABCD), Amphotericin B lipid complex (ABLC), Amphotericin B liposomal, Caspofungin acetate, Clotrimazole, Fluconazole, Flucytosine, Griseofulvin, Itraconazole, Ketoconazole, Miconazole, Nystatin, Pentamidine, Terbinafine, and Voriconazole.

In some embodiments, methods encompassed by this disclosure further include administration of an antiviral drug. Antiviral drugs include, but are not limited to, Abacavir, Aciclovir, Amantadine, Didanosine, Emtricitabine, Enfuvirtide, Entecavir, Lamivudine, Nevirapine, Ribavirin, Rimantidine, Stavudine, Valaciclovir, Vidarabine, Zalcitabine, and Zidovudine.

### IV. EXEMPLARY PHARMACEUTICAL COMPOSITIONS

The disclosure also pertains to pharmaceutical compositions including one or more compounds used in the methods described herein and a pharmaceutically acceptable diluent or carrier. Such pharmaceutical compositions may be included in a kit or container. Such kit or container maybe packaged with instructions pertaining to the method of treating a disease, as described herein. Such compositions may be used in methods of curing, treating, preventing, or ameliorating a disease or a disease symptom in a patient, preferably a mammal and most preferably a human, by using the methods described herein. The compositions described herein may also comprise a combination of lymphocytoxic non-myeloablative amount of an oxazaphosphorine drug and at least one other agent, for example, granulocyte colony stimulating factor and an antimicrobial agent. Also provided herein is a composition comprising a combination of lymphocytoxic non-myeloablative amount of an oxazaphosphorine drug and at least one other agent, wherein platelets are administered prior to, simultaneously, or following administration of the oxazaphosphorine composition.

For example, in some embodiments, encompassed by this disclosure is a kit for treating an immune disorder chosen from an autoimmune disease, an allergic reaction and transplant rejection including: (a) a plurality of doses of a non-myeloablative oxazaphosphorine drug; and (b) instructions for treating the immune disorder using one or more doses of the oxazaphosphorine drug; wherein the one or more doses are lymphocytotoxic.

In some embodiments, a kit for treating an immune disorder chosen from an autoimmune disease, an allergic reaction and transplant rejection further includes one or more of: (a) a plurality of doses of granulocyte colony stimulating factor; (b) a plurality of doses of platelets; and (d) a plurality of doses of one or more antimicrobial agent.

In further embodiments, kits encompassed by this disclosure include instructions for using the kit to treat an immune disorder chosen from an autoimmune disease, an allergic reaction and transplant rejection.

### V. MODES OF ADMINISTRATION

The various compounds used in the methods described herein may be administered orally, parenterally (e.g., intravenously), intramuscularly, sublingually, buccally, rectally, intranasally, intrabronchially, intrapulmonarily, intraperitonealy, topically, transdermally and subcutaneously, for example. The amount of compound administered in a single dose may dependent on the subject being treated, the subject's weight, the manner of administration and the judgment of the prescribing physician. Generally, however, administration and dosage and the duration of time for which a composition is administered will approximate that which are necessary to achieve a desired result, for example, at least a single dose of 5 µg/kg of granulocyte colony stimulating factor for increasing neutrophil count to a level which falls within a normal range. Generally, GCSF is administered at 5 micrograms/kg/daily starting 6 days after the last dose of cyclophosphamide and is continued the drug until the absolute neutrophil count reaches 1000. In some embodiments, NEULASTA® is administered in place of GCSF.

For example, in some embodiments, a lymphocytotoxic non-myeloablative amount of a oxazaphosphorine drug used in the methods described herein is between 100 mg/kg and 200 mg/kg, administered daily from 1 to 7 days. In certain embodiments, an effective amount of a lymphocytotoxic non-myeloablative amount of a oxazaphosphorine drug is between 25 mg/kg and 100 mg/kg, administered daily for 2 to 6 consecutive days or administered daily for 3 to 5 consecutive days, for example 4 consecutive days. In certain embodiments, a lymphocytotoxic non-mycloablative amount of a oxazaphosphorine drug is about 50 mg/kg administered daily for 4 consecutive days. In certain embodiments, a lymphocytotoxic non-myeloablative amount of a oxazaphosphorine drug is 50 mg/kg administered daily for 4 consecutive days.

In some embodiments, an effective amount of platelets are administered to a subject in need thereof for a duration of time necessary for the platelet count to be, for example, between 100,000 platelets/mm³ and 110,000 platelets/mm³, or between 110,000 platelets/ mm³ and 120,000 platelets/mn³, or between 120,000 platelets/mm³ and 130,000 platelels/mm³, or greater than 130,000 platelets/mm³. In some embodiments, platelets are administered to a subject in need thereof, for a duration of time necessary for the platelet count to be at least 10,000 platelets/mn³.

In some embodiments, an effective amount of granulocyte colony stimulating factor is administered for a duration of time necessary for the neutrophil count to be at least 500/mm³, or at least 1000/mm³, or at least 1500/mm³, or greater than 1500/mm³_{.}

In some methods encompassed by this disclosure, an effective amount of granulocyte colony stimulating factor is 5 µg/kg/mg/day, which is administered for a duration of time necessary for the neutrophil count to be at least 1000/mm³.

The optimal dosages for administration include those described herein and those, which may be routinely determined by a skilled artisan using well-known techniques.

Depending on the intended mode of administration, the compounds used in the methods described herein may be in the form of solid, semi-solid or liquid dosage forms, such as, for example, tablets, suppositories, pills, capsules, powders, liquids, suspensions, lotions, creams, gels, or the like, preferably in unit dosage form suitable for single administration of a precise dosage. Each dose may include an effective amount of a compound used in the methods described herein in combination with a pharmaceutically acceptable carrier and, in addition, may include other medicinal agents, pharmaceutical agents, carriers, adjuvants, diluents, etc.

Liquid pharmaceutically administrable compositions can prepared, for example, by dissolving, dispersing, etc., a compound for use in the methods described herein and optional pharmaceutical adjuvants in an excipient, such as, for example, water, saline aqueous dextrose, glycerol, ethanol, and the like, to thereby form a solution or suspension. For solid compositions, conventional nontoxic solid carriers include, for example, pharmaceutical grades of mannitol, lactose, starch, magnesium stearate, sodium saccharin, talc, cellulose, glucose, sucrose, magnesium carbonate, and the like. If desired, the pharmaceutical composition to be administered may also contain minor amounts of nontoxic auxiliary substances such as wetting or emulsifying agents, pH buffering agents and the like, for example, sodium acetate, sorbitan monolaurate, triethanolamine sodium acetate, triethanolamine oleate, etc. Actual methods of preparing such dosage forms are known, or will be apparent, to those skilled in this art; see, for example, Remington's Pharmaceutical Sciences, 18th Ed. (1990), Mack Publishing Co., Easton, Pa., the entire disclosure of which is hereby incorporated by reference).

### VI. METHODS OF TREATMENT

Methods of treatment described herein encompass methods of treating an immune disorder including an autoimmune disease, an allergic reaction and transplant rejection by, for example, reconstituting a subject's immune system. Also encompassed are methods of eliminating immune cells, which arc capable of eliciting an adverse immune reaction in a subject. Certain methods described herein exclude the use of autologous or allogeneic stem cell transplantation and/or additional immunomodulatory agents.

Accordingly, in some embodiments, this disclosure provides a method of treating an immune disorder other than severe aplastic anemia, chronic inflammatory demyelinating polyneuropathy, paraneoplastic pemphigus, pemphigus foliaceus, pemphigus vulgaris, or systemic lupus erythematosus in a subject including administering a lymphocytotoxic non-myeloablative amount of a oxazaphosphorine drug to the subject, where the method does not include the use of both stem cell transplantation and additional immunomodulatory agents, and where there is no relapse for at least 1 year. In certain embodiments, the disclosure provides a method of treating an immune disorder, in a subject including administering a lymphocytotoxic non-mycloablative amount of a oxazaphosphorine drug to the subject, where the method does not include the use of both stem cell transplantation and additional immunomodulatory agents, and where there is no relapse for at least 4 years.

Methods of treating an immune disorder other than severe aplastic anemia may additionally include one or more of the steps of (a) administering an effective amount of granulocyte colony stimulating factor to the subject; (b) administering an effective amount of an antimicrobial agent to the subject; (c) administering an effective dose of platelets to the subject, and any combinations thereof.

In certain embodiments, the disclosure provides methods of treating an immune disorder including: AIDS-associated myopathy, AIDS-associated neuropathy, Acute disseminated encephalomyelitis, Addison's Disease, Alopecia Areata, Anaphylaxis Reactions, Ankylosing Spondylitis, Antibody-related Neuropathies, Antiphospholipid Syndrome, Autism, Autoimmune Atherosclerosis, Autoimmune Diabetes Insipidus, Autoimmune Endometriosis, Autoimmune Eye Diseases, Autoimmune Gastritis, Autoimmune Hemolytic Anemia, Autoimmune Hemophilia, Autoimmune Hepatitis, Autoimmune Interstitial Cystitis, Autoimmune Lymphoproliferative Syndrome, Autoimmune Myelopathy, Autoimmune Myocarditis, Autoimmune Neuropathies, Autoimmune Oophoritis, Autoimmune Orchitis, Autoimmune Thrombocytopenia, Autoimmune Thyroid Diseases, Autoimmune Urticaria, Autoimmune Uveitis, Autoimmune Vasculitis, Behcet's Disease, Bell's Palsy, Bullous Pemphigoid, CREST, Celiac Disease, Cerebellar degeneration (paraneoplastic), Cluronic Fatigue Syndrome, Chronic Rhinosinusitis, Chronic inflammatory demyclinating polyneuropathy, Churg Strauss Syndrome, Connective Tissue Diseases, Crohn's Disease, Cutaneous Lupus, Dermatitis Herpetiformis, Dermatomyositis, Diabetes Mellitus, Discoid Lupus Erythematosus, Drug-induced Lupus, Endocrine Orbitopathy, Glomerulonephritis, Goodpasture Syndrome, Goodpasture's Syndrome, Graves Disease, Guillain-Barre Syndrome, Guillian Barre Syndrome (Miller Fisher variant), Guillian Barre Syndrome (axonal), Guillian Barre Syndrome (demyelinating), Hashimoto's Thyroiditis, Herpes Gestationis, Human T-cell lymphomavirus-associated myelopathy, Huntington's Disease, IgA Nephropathy, Immune Thrombocytopenic Purpura, Inclusion body myositis, Interstitial Cystitis, Isaacs syndrome, Lambert Eaton myasthenic syndrome, Limbic encephalitis, Lower motor neuron disease, Lyme Disease, MCTD, Microscopic Polyangiitis, Miller Fisher Syndrome, Mixed Connective Tissue Disease, Mononeuritis multiplex (vasculitis), Multiple Sclerosis, Myasthenia Gravis, Myxedema, Meniere Disease, Neonatal LE, Neuropathies with dysproteinemias, Opsoclonus-myoclonus, PBC, POEMS syndrome, Paraneoplastic Autoimmune Syndromes, Pemphigus, Pemphigus Foliaceus, Pemphigus Vulgaris, Pernicious Anemia, Peyronie's Disease, Plasmacytoma/myeloma neuropathy, Poly-Dermatomyositis, Polyarteritis Nodosa, Polyendocrine Deficiency Syndrome, Polyendocrine Deficiency Syndrome Type 1, Polyendocrine Deficiency Syndrome Type 2, Polyglandular AutoimmuneSyndrome Type I, Polyglandular Autoimmune Syndrome Type 11, Polyglandular Autoimmune Syndrome Type III, Polymyositis, Primary Biliary Cirrhosis, Primary Glomerulonephritis, Primary Sclerosing Cholangitis, Psoriasis, Psoriatic Arthritis, Rasmussen's Encephalitis, Raynaud's Disease, Relapsing Polychondritis, Retrobulbar neuritis, Rheumatic Diseases, Rheumatoid Arthritis, Scleroderma, Sensory neuropathies (para neo plastic), Sjogren's Syndrome, Stiff-Person Syndrome, Subacute Thyroiditis, Subacute autonomic neuropathy, Sydenham Chorea, Sympathetic Ophthalmitis, Systemic Lupus Erythematosus, Transverse myelitis, Type 1 Diabetes, Ulcerative Colitis, Vasculitis, Vitiligo, Wegener's Granulomatosis, Acrocyanosis, Anaphylactic reaction, Autoimmune inner ear disease, Bilateral sensorineural hearing loss, Cold agglutinin hemolytic anemia, Cold-induced immune hemolytic anemia, Idiopathic endolymphatic hydrops, Idiopathic progressive bilateral sensorineural hearing loss, Immune-mediated inner ear disease, and Mixed autoimmune hemolysis, but not including aplastic anemia, in a subject comprising administering (a) a lymphocytotoxic non-myeloablative amount of a oxazaphosphorine drug to the subject, where the method does not include the use of both stem cell transplantation and additional immunomodulatory agents, and wherein the methods may further comprise (b) administering an effective amount of granulocyte colony stimulating factor to the subject; (c) administering an effective amount of at least one antimicrobial agent to the subject; and (d) administering an effective amount of platelets to the subject.

In further embodiments, this disclosure provides a method for eliminating or substantially reducing an immune disorder in a subject other than aplastic anemia, chronic inflammatory demyelinating polyneuropathy, myasthenia gravis, paraneoplastic pemphigus, pemphigus foliaceus, or systemic lupus erythematosus comprising administering a lymphocytotoxic non-myeloablative amount of a oxazaphosphorine drug to the subject, such that the subject's immune system reconstitutes without stem cell transplantation. In certain embodiments, the oxazaphosphorine drug is cyclophosphamide. Cyclophosphamide may be administered to the subject at 50 mg/kg for 4 consecutive days. In further embodiments, the method may further comprise (a) administering to the subject an effective amount of granulocyte colony stimulating factor; (b) administering to the subject an effective amount of platelets; and (c) administering to the subject an effective amount of at least one antimicrobial agent, such that the immune disorder other than aplastic anemia, chronic inflammatory demyelinating polyneuropathy, myasthenia gravis, paraneoplastic pemphigus, pemphigus foliaceus, or systemic lupus erythematosus is treated in the subject, and/or where the method does not include both stem cell transplantation and/or administration of additional immunomodulatory agents.

In further embodiments, this disclosure relates to a method of obtaining a cell population substantially free of cells capable of eliciting an adverse immune reaction in a subject including: (a) administering a lymphocytotoxic non-myeloablative amount of a oxazaphosphorine drug to the subject, followed by (b) administering an effective amount of granulocyte colony stimulating factor to the subject; (c) administering an effective amount of at least one antimicrobial agent to the subject; and (d) administering an effective amount of platelets to the subject, where the method does not include the use of both stem cell transplantation and additional immunomodulatory agents.

In addition to autoimmune diseases, this disclosure also encompasses the treatment of other adverse immune reactions such as allergic reactions and transplant rejections. The method comprises administering (a) a lymphocytotoxic non-myeloablative amount of a oxazaphosphorine drug to the subject, where the method does not include the use of both stem cell transplantation and additional immunomodulatory agents, and wherein the method may further comprise (b) administering an effective amount of granulocyte colony stimulating factor to the subject; (c) administering an effective amount of at least one antimicrobial agent to the subject; and (d) administering an effective amount of platelets to the subject.

In some embodiments, one or more methods described herein further include the step of identifying a subject with the immune disorder, which is being treated using a method described herein.

In some embodiments, this disclosure relates to the treatment of scleroderma in a subject comprising administration of a lymphocytotoxic non-myeloablative amount of a oxazaphosphorine drug to the subject, thereby to treat scleroderma.

In some embodiments, this disclosure provides a method of treating multiple sclerosis in a subject in need thereof comprising: (a) identifying a subject that failed to respond to conventional therapy; and (b) administering a lymphocytotoxic non-myeloablative amount of a oxazaphosphorine drug to the subject, thereby to treat multiple sclerosis. In some embodiments, this disclosure provides a method of treating multiple sclerosis in a subject comprising: (a) identifying a subject having at least two gadolinium enhancing lesions; and (b) administering a lymphocytotoxic non-myeloablative amount of a oxazaphosphorine drug to the subject, thereby to treat multiple sclerosis. In certain embodiments, the multiple sclerosis is aggressive relapsing remitting multiple sclerosis. In certain embodiments, the oxazaphosphorine drug is cyclophosphamide. Cyclophosphamide may be administered to the subject at 50 mg/kg for 4 consecutive days. In further embodiments, the method may further comprise (a) administering to the subject an effective amount of granulocyte colony stimulating factor; (b) administering to the subject an effective amount of platelets; and (c) administering to the subject an effective amount of at least one antimicrobial agent, such that multiple sclerosis is treated in the subject, and/or where the method does not include stem cell transplantation and/or administration of additional immunomodulatory agents.

In some embodiments, this disclosure provides a method of treating systemic lupus erythematosus in a subject in need thereof including: (a) administering to the subject 50 mg/kg of cyclophosphamide for 4 consecutive days followed by, (b) administering to the subject an effective amount of granulocyte colony stimulating factor; (c) administering to the subject an effective amount of platelets; and (d) administering to the subject an effective amount of at least one antimicrobial agent, such that systemic lupus erythematosus is treated in the subject, where the method does not include both stem cell transplantation and administration of additional immunomodulatory agents.

In certain embodiments, this disclosure provides a method of treating autoimmune hemolytic anemia in a subject in need thereof comprising: (a) administering to the subject 50 mg/kg of cyclophosphamide for 4 consecutive days followed by, (b) administering to the subject an effective amount of granulocyte colony stimulating factor, such that autoimmune hemolytic anemia is treated in the subject, where the method does not include both stem cell transplantation and administration of additional immunomodulatory agents.

Also provided is a method of treating autoimmune thrombocytopenia in a subject in need thereof comprising: (a) administering to the subject 50 mg/kg of cyclophosphamide for 4 consecutive days followed by, (b) administering to the subject an effective amount of granulocyte colony stimulating factor; (c) administering to the subject an effective amount of platelets; and (d) administering to the subject an effective amount of at least one antimicrobial agent, such that autoimmune thrombocytopenia is treated in the subject, where the method does not include both stem cell transplantation and administration of additional immunomodulatory agents.

In some embodiments, this disclosure includes a method of treating pemphigus vulgaris in a subject in need thereof comprising: (a) administering to the subject 50 mg/kg of cyclophosphamide for 4 consecutive days followed by, (b) administering to the subject an effective amount of granulocyte colony stimulating factor; (c) administering to the subject an effective amount of platelets; and (d) administering to the subject an effective amount of at least one antimicrobial agent, such that pemphigus vulgaris is treated in the subject, where the method does not include both stem cell transplantation and administration of additional immunomodulatory agents.

Also encompassed by this disclosure is a method of treating myasthenia gravis in a subject in need thereof comprising: (a) administering to the subject 50 mg/kg of cyclophosphamide for 4 consecutive days followed by, (b) administering to the subject an effective amount of granulocyte colony stimulating factor; (c) administering to the subject an effective amount of platelets; and (d) administering to the subject an effective amount of at least one antimicrobial agent, such that myasthenia gravis is treated in the subject, where the method docs not include both stem cell transplantation and administration of additional immunomodulatory agents.

### EXAMPLES

The invention having been generally described, may be more readily understood by reference to the following examples, which are included merely for purposes of illustration of certain aspects and embodiments of the present invention, and are not intended to limit the invention in any way.

### Example 1: Use of high dose cyclophosphamide for the treatment of multiple sclerosis

High-dose cyclophosphamide is used for treating multiple sclerosis, including aggressive relapsing remitting multiple sclerosis (MS). MS is an autoimmune disease characterized by progressive immune-mediated destruction of myelin and axons within the CNS. At least five conventional therapies are described for the treatment of MS including, for example, interferon β-1b (BETASERON), interferon β-1a (AVONEX and REBIF), glatiramer acetate (COPAXONE) and mitixantrone (NOVANTRONE). High-dose cyclophosphamide therapy is especially useful for the treatment of those MS patients that fail to respond to conventional therapy. Such patients are identified, for example, by the Expanded Disability Status Scale (EDSS), the MS functional composite (MSFC), neurocognitive studies and brain parenchymal fraction (BPF).

Most patients on conventional immunomodulatory therapy continue to accrue progressive disability. Although immunoablation strategies with transplantation may be effective in some patients in halting disease and inducing stable remission, these strategies are associated with unacceptable mortality rates, precluding the use of this treatment in most patients. Additionally, long-term conventional immunomodulatory treatment and immune ablation with transplantation are exceedingly expensive therapies and may result in only temporary disease suppression.

Patients that do not respond to a conventional therapy, as identified by one or more of the foregoing criteria (e.g., having an EDSS from 1.5-6.5), are treated with high-dose cyclophosphamide (e.g., 50 mg/kg/3-4 days) followed by administration of GCSF, 6 days after the completion of high-dose cyclophosphamide treatment, until the neutrophil count exceeds 1.0 X 10⁹ per liter. Patients are typically administered antibiotics until their neutrophil count returns to within the normal range.

High-dose cyclophosphamide treatment is also used for treating patients that have at least one, or at least two gadolinium enhancing lesions in the brain. Such lesions can be identified, for example, using MRI and other brain scanning techniques.

As described herein, we have also investigated the use and safety of high-dose cyclophosphamide without transplantation in patients with aggressive MS. Open-label trial of patients with aggressive MS were given an up-front regimen of 50 mg/kg/d for four consecutive days. Enrolled patients had aggressive MS as defined by 2 or more total gadolinium enhancing lesions on each oftwo pretreatment MRI scans; at least one clinical exacerbation in the last year despite being on conventional MS therapy; and sustained increase of ≥ 1.0 on the EDSS in the preceding year.

Eight patients completed the cyclophosphamide administration and no patients demonstrated an unexpected grade 3 or 4 adverse event. All patients developed transient severe neutropenia, an expected consequence, followed by immune reconstitution in 10-17 days. All patients demonstrated a reduction or elimination of new and enhancing lesions on the MRI. Brain atrophy has been slowed in several patients. No patient demonstrated a clinical exacerbation following treatment and most patients showed a reduction in EDSS and an improvement in the MSFC following treatment. We also analyzed changes in microglial activation after HiCy using [¹¹C]-R-PK11195-PET imaging.

High-dose cyclophosphamide treatment is safer and more effective than using immunoablation with stem cell transplantation for treating MS, including aggressive MS.

### Example 2: High-dose Cyclophosphamide Treatment of Severe Aplastic Anemia

Acquired severe aplastic anemia (SAA) is a rare hematopoietic disorder characterized by pancytopenia and a hypocellular bone marrow. With supportive care alone, most patients die of the disease within a year of diagnosis. The majority of acquired SAA results from autoimmune destruction of bone marrow cells. Like other autoimmune diseases damage to the target organ (i.e., the bone marrow) is felt to be mediated by cytotoxic T-lymphocytes, which are demonstrable in the blood and marrow.

The pathophysiology of aplastic anemia has led to two main approaches to therapy: replacement of both the immune system and deficient hematopoictic stem cells by allogeneic SCT (in patients with a suitable donor) or suppression of the destructive immunologic process with anti-thymocyte globulin and cyclosporine (ATG/CSA). Allogeneic SCT from an HLA identical sibling has the potential to cure SAA. In patients under the age of 25 the cure rate is 80-90%; however, in patients older than 40 years, the cure rate is roughly 50%. For patients with no HLA identical sibling ATG/CSA is commonly employed. ATG/CSA leads to improved hemopoiesis in 60-80% of patients, but does not often result in cure. Most patients relapse, become dependent on long-term immunosuppression or acquire a secondary clonal disease, such as paroxysmal nocturnal hemoglobinuria (PNH) or myelodysplastic syndromes (MDS).

In a study of 10 SAA patients treated with high-dose cyclophosphamide, complete remission (normocellular bone marrow, hemoglobin > 13.0 g/dl, neutrophil count > 1.5 x 10⁹/L and a platelet count greater than 125 x 10⁹/L) was achieved in seven of ten patients. In a further study, an additional 19 previously untreated SAA patients (median age, 47 years) were treated with high-dose cyclophosphamide. The probability of survival was 84% (95% C1, 59- 95%) at 24 months. The probability of achieving treatment-free remission was 73% (95% CI, 51- 91%). No responding patients have had relapse or have developed secondary clonal disorders. The median time to a neutrophil count of 500 µL was 49 days.

Further, we treated 38 previously untreated, and 17 immunosuppressive therapy failed, SAA patients with high-dose cyclophosphamide (50 mg/kg/d x 4) followed by daily GCSF (5 ug/kg/day) until the neutrophil count (ANC) reached 1000/dl. Response was defined as ANC > 1000/dl and transfusion independence without growth factor support for > 3 months. Relapse was defined as no longer meeting criteria for response. Development of paroxysmal nocturnal hemoglobinuria was monitored by flow cytometry.

The median age of the newly diagnosed patients was 40 (range 2-68) years. With a median follow-up of 41 (range, 6 ― 111) months, 33/38 patients survive (actuarial survival of 86%, 95% CI 72-95%) with 28 (74%, 95% CI 58-85%) achieving remission, most being complete. Median time to ANC of 500, last platelet and red cell transfusion was 50, 99, and 181 days, respectively. Before treatment, 15 patients met criteria for very (v) SAA (ANC < 200). Mortality within 6 months after high-dose cyclophosphamide treatment occurred in 4 (10.5%) patients, all with vSAA; 1 additional patient died from bacterial sepsis 18 months after high-dose cyclophosphamide treatment. 22/23 (96%) SAA patients survive (20 in remission) compared to 11/15 (73%) with SAA (10 in remission). Eight patients had a severe infection at the time of beginning treatment and 5 survive in remission. PNH screening revealed a PNH population ranging from 0.5-40% of granulocytes in 12 patients, and all 12 achieved a durable remission (p = 0.039). No patient in this series has progressed to PNH or MDS, and the PNH clone is regressing in all 12 patients. Two patients have relapsed. One patient, whose first remission lasted 5 years, was retreated with high-dose cyclophosphamide into a persisting second complete remission 3 years ago; another patient recently relapsed 3 years after achieving remission. Ten of the 17 patients who failed immunosuppressive therapy (median age of 31, range 6-58) are alive and nine are in remission.

High-dose cyclophosphamide is a safe and highly effective therapy for both untreated and relapsed SAA. Relapses after high-dose cyclophosphamide are rare and progression to paroxysmal nocturnal hemoglobinuria or myelodysplastic syndromes in previously untreated patients has not been observed in this series with now 15 patients out beyond 5 years. The presence of a paroxysmal nocturnal hemoglobinuria population may be a favorable risk factor, perhaps by excluding non-immune mediated forms of SAA.

### Example 3: Treatment of Hepatitis-Associated Aplastic Anemia with High Dose Cyclophosphamide

Hepatitis-associated aplastic anemia (HAA) is a rare variant ofaplastic anemia that accounts for 5% of cases. The hepatitis is seronegative and most often spontaneously resolves. The aplastic anemia that follows presents within a few months after the onset of hepatitis and is often fatal. One study that investigated the treatment for HAA used antithymocyte globulin and cyclosporine, which induced remissions in 7 of 10 patients, with up to one year of follow-up. In that study, there were 3 deaths related to treatment failure and 1 relapse. High-dose cyclosphosphamide induces durable remissions in severe aplastic anemia (SAA) and other autoimmune diseases, and we hypothesized that it could induce durable remissions in HAA as well.

Five patients (ages 6-17 years) with HAA and without a matched sibling BMT option were treated with cyclophosphamide (50 mg/kg/day IV x 4 days) plus mesna. Serology/PCR for HAV, HBV, HCV, EBV, and CMV were negative. All patients met criteria for very severe aplastic anemia pretreatment: bone marrow cellularity <25%, ANC <200µL, platelet count <20,000/µL, absolute reticulocyte count <60,000/µL. Infection prophylaxis consisted of trimethoprim/sulfamethoxazole, G-CSF, and fluconazolc.

All patients were transfusion dependent for erythrocytes and platelets prior to high dose cyclophosphamide. Other baseline and current values are shown in Table I. Four patients demonstrated hematopoietic recovery. Median time to ANC>500/µL was 51 days (range 44-369). Median time to transfusion independence for erythrocytes and platelets was 109 (range 57-679) and 160 (range 48-679) days, respectively. The 4 patients with hematopoietic responses are in remission up to 6 years after treatment without further immune suppression beyond high-dose cyclophosphamide. Patient 2 met criteria for autoimmune hepatitis (AH-1), and her AIH remains in remission, as well. Patient 5 had no hematopoietic response and died 3 months after BMT of multi-organ failure.

High-dose cyclophosphamide induced durable remissions in hepatitis-associated aplastic anemia in 4 of 5 patients based on follow-up from 1-6 years. Treatment failure led to one death in this series. The remission of HAA and AIH in one patient suggests that high dose CY may be an alternative and effective treatment for AIH, which is a disease characterized by long-term dependence on immunosuppression and recurrentrelapses.

**Table I: Pre-Treatment and Current Patient Characteristics**

| Age/Sex | Follow Up | ANC (cells/µL) | ALT (U/L) | High (g/dL) | Platelets (1000/µL) |
|---|---|---|---|---|---|
| | | Nadir/Current | Peak/Current | Current | Current |
| 17/F | 6y | 138/3430 | 1832/16 | 11.1 | 99 |
| 9/F* | 2y | 178/2500 | 1186/11 | 13 | 140 |
| 14/M 6/F | 16mo 13 mo | 0/2380 0/669 | 2800/31 2213/21 | 13.7 13.9 | 98 37 |
| 16/F | 5mo† | 0/† | 3051/† | † | † |

| | | | | | |
|---|---|---|---|---|---|
| *ANA titer>1:640 and anti-smooth muscle Ab titer 1:40. †Had no hematologic response and proceeded to unrelated BMT after 5 months. | | | | | |

### Example 4: High-dose Cyclophosphamide Treatment for other Refractory Autoimmune Disorders

To investigate the treatment of other autoimmune and possibly alloimmune conditions using high-dose cyclophosphamide, eight patients suffering from a variety of severe refractory autoimmune disorders (2 systemic lupus erythematosus, 2 Felty syndrome, 1 immune thrombocytopenia, 2 autoimmune hemolytic anemia, and 1 chronic inflammatory demyelinating polyneuropathy) were treated with high-dose cyclophosphamide. Seven patients showed marked clinical improvement: five achieved a complete remission and 2 achieved a partial remission. Hematopoietic reconstitution was rapid. The median time to a neutrophil count of 500 per µL and platelet transfusion independence was 17 and 16 days after the last dose of cyclophosphamide, respectively.

In a further study, 14 patients with moderate to severe systemic lupus erythematosus (SLE) that was refractory to corticosteroids and one or more additional immunosuppressive regimen were treated with high-dose cyclophosphamide. In this group, the median time to a neutrophil count of 500 µl was 14 days (range, 11 to 22 days) after the last dose of cyclophophamide. Patients required a median of 2 transfusions (range, 2 to 5) of packed red blood cells and the median day to last platelet transfusion was day 16 (range, 0 to 23). There were no deaths or fungal infections. A significant improvement in Physicians Global Assessment (mean difference 1.3, p≤0.0001), systemic lupus erythematosus disease activity index (mean difference 3.5 p=0.042) and prednisone dosage (mean difference 12.8 mg, p=0.01) was observed. Responses, including 5 durable complete responses, were observed in all organ systems, renal, central nervous system and skin that led to patient enrollment.

High-dose cyclophosphamide also induces durable complete remissions in patients with parancoplastic pemphigus and pemphigus vulgaris. Following high-dose cyclophosphamide treatment, a patient with paraneoplastic pemphigus did not require blood products and recovered to a neutrophil count of greater than 500 per µL by day 15. The patient with pemphigus vulgaris began to recover neutrophils by day 9; he received 2 platelet transfusions, but did not require red cell transfusions. In both patients, the pathogenic autoantibodies specific for the disease became undetectable after high-dose cyclophosphamide treatment.

Durable remission following high-dose cyclophosphamide was also observed in refractory autoimmune hemolytic anemia. For example, 9 patients suffering from refractory autoimmune hemolytic anemia were treated with high-dose cyclophosphamide; 7 had an IgG warm autoantibody, one had an IgM cold agglutinin and one had both warm and cold agglutinin disease. The median hemoglobin at the time of treatment was 6.7 (range; 5-10) g/dl and 8 of the 9 patients were dependent on erythrocyte transfusions. The median times to a neutrophil count of 500 per µL and to platelet transfusion independence after high-dose cyclophosphamide treatment was 16 and 15 days, respectively. All patients responded and became transfusion independent; 6 patients achieved a complete remission (normal untransfused hemoglobin for age and sex) and 3 patients achieved a partial remission (hemoglobin > 10.0 g/dl without support of transfusions). There were no relapses at a median follow-up of 15 (range; 4-29) months and 7 of the 9 patients were able to discontinue steroids.

High-dose cyclophosphamide may also be used to eradicate alloimmunization, a major problem in patients who require chronic blood transfusions and in patients being considered for organ transplantation. Five patients with SAA who were refractory to platelet transfusions due to HLA-specific antibodies were studied before and after treatment with high-dose cyclophosphamide. Complete remission of the SAA was achieved in four of these five patients. All four responders demonstrated a marked reduction in anti-HLA antibody titer after high-dose cyclophosphamide; in three of these patients the antibody was completely eradicated suggesting that high-dose cycloptiosphamide may have the potential to treat alloimmune conditions.

In a further study, we treated 7 patients with myasthenia gravis refractory to extensive conventional immunosuppressive therapy, using high-dose cyclophosphamide. All of these patients markedly improved, and returned to full activity.

The specification is most thoroughly understood in light of the teachings of the references cited within the specification, which arc hereby incorporated by reference. The embodiments within the specification provide an illustration of embodiments in this disclosure and should not be construed to limit its scope. The skilled artisan readily recognizes that many other embodiments are encompassed by this disclosure. All publications and patents cited and sequences identified by accession or database reference numbers in this disclosure are incorporated by reference in their entirety. To the extent that the material incorporated by reference contradicts or is inconsistent with the present specification, the present specification will supercede any such material. The citation of any references herein is not an admission that such references are prior art to the present disclosure.

Unless otherwise indicated, all numbers expressing quantities of ingredients, cell culture, treatment conditions, and so forth used in the specification, including claims, are to be understood as being modified in all instances by the term "about." Accordingly, unless otherwise indicated to the contrary, the numerical parameters are approximations and may vary depending upon the desired properties sought to be obtained by the present invention. Unless otherwise indicated, the term "at least" preceding a series of elements is to be understood to refer to every element in the series. Those skilled in the art will recognize, or be able to ascertain using no more than routine experimentation, many equivalents to the specific embodiments of the invention described herein. Such equivalents are intended to be encompassed by the following claims.

## Claims

1. Use of a lymphocytoxic non-myeloablative amount of a oxazaphosphorine drug for the preparation of a medicament for (a) treating a subject having an immune disorder excluding aplastic anemia, chronic inflammatory demyelinating polyneuropathy, paraneoplastic pemphigus, pemphigus foliaceus, pemphigus vulgaris or systemic lupus erythematosus, such that the subject's immune system reconstitutes without stem cell transplantation and optionally such that the disease remains in remission without administration of additional immunomodulatory agents, and wherein there is no relapse for at least 1 year; or (b) treating a subject having an immune disorder, such that the subject's immune system reconstitutes without stem cell transplantation and such that the disorder remains in remission without the administration of additional immunomodulatory agents, and wherein there is no relapse for at least 4 years; optionally wherein there is no relapse for at least 5 years; optionally wherein there is no relapse for at least 10 years; or (c) treating a subject having an immune disorder, such that the subject's immune system reconstitutes without stem cell transplantation and such that the disorder remains in remission without the administration of additional immunomodulatory agents, and wherein the treatment comprises a cure of the immune disorder.

2. The use of claim 1 (a) or 1 (b), wherein treating the subject further comprises one or more steps selected from the group consisting of:
(a) administering an effective amount of granulocyte colony stimulating factor to the subject;
(b) administering an effective amount of at least one antimicrobial agent to the subject; and
(c) administering an effective amount of platelets to the subject; optionally wherein (i) an effective amount of platelets is an amount which results in a platelet count of at least 125,000 platelets/mm³; or (ii) an effective amount of granulocyte colony stimulating factor is an amount which results in a neutrophil count of at least 500/mm³, e.g., wherein an effective amount of the granulocyte colony stimulating factor is 5 µg/kg/day.

3. Use of a lymphocytoxic non-myeloablative amount of a oxazaphosphorine drug in the preparation of a medicament to treat a subject having an immune disorder in the absence of stem cell transplantation and without administration of additional immunomodulatory agents, and wherein treating the subject may further comprise:
(a) administering an effective amount of granulocyte colony stimulating factor to the subject; and
(b) administering an effective amount of at least one antimicrobial agent to the subject.

4. The use of claims 1 (a) or 1 (b) or 3, wherein the immune disorder is selected from the group consisting of: an autoimmune disease, an allergic reaction, and transplant rejection; optionally wherein:
(a) the autoimmune disease is selected from the group consisting of: AIDS-associated myopathy, AIDS-associated neurophathy, Acute disseminated encephalomyelitis, Addison's Disease, Alopecia Areata, Anaphylaxis Reactions, Ankylosing Spondylitis, Antibody-related Neuropathies, Antiphospholipid Syndrome, Autism, Autoimmune Atherosclerosis, Autoimmune Diabetes Insipidus, Autoimmune Endometriosis, Autoimmune Eye Diseases, Autoimmune Gastritis, Autoimmune Hemolytic Anemia, Autoimmune Hemophilia, Autoimmune Hepatitis, Autoimmune Interstitial Cystitis, Autoimmune Lym pho proliferative Syndrome, Autoimmune Myelopathy, Autoimmune Myocarditis, Autoimmune Neuropathies, Autoimmune Oophoritis, Autoimmune Orchitis, Autoimmune Thrombocytopenia, Autoimmune Thyroid Diseases, Autoimmune Urticaria, Autoimmune Uveitis, Autoimmune Vasculitis, Behcet's Disease, Bell's Palsy, Bullous Pemphigoid, CREST, Celiac Disease, Cerebellar degeneration (paraneoplastic), Chronic Fatigue Syndrome, Chronic Rhinosinusitis, Chronic inflammatory demyelinating polyneuropathy, Churg Strauss Syndrome, Connective Tissue Diseases, Crohn's Disease, Cutaneous Lupus, Dermatitis Herpetiformis, Dermatomyositis, Diabetes Mellitus, Discoid Lupus Erythematosus, Drug-induced Lupus, Endocrine Orbitopathy, Glomerulonephritis, Goodpasture Syndrome, Goodpasture's Syndrome, Graves Disease, Guillian-Barre Syndrome, Miller Fisher variant of the Guillian Barre Syndrome, axonal Guillian Barre Syndrome, demyelinating Guillian Barre Syndrome, Hashimoto Thyroiditis, Herpes Gestationis, Human T-cell lymphomavirus-associated myelopathy, Huntington's Disease, IgA Nephropathy, Immune Thrombocytopenic Purpura, Inclusion body myositis, Interstitial Cystitis, Isaacs syndrome, Lambert Eaton myasthenic syndrome, Limbic encephalitis, Lower motor neuron disease, Lyme disease, MCTD, Microscopic Polyangiitis, Miller Fisher Syndrome, Mixed Connective Tissue Disease, Mononeuritis multiplex (vasculitis), Multiple Sclerosis, Myasthenia Gravis, Myxedema, Meniere Disease, Neonatal LE, Neuropathies with dysproteinemias, Opsoclonus-myoclonus, PBC, POEMS syndrome, Paraneoplastic Autoimmune Syndromes, Pemphigus, Pemphigus Foliaceus, Pemphigus Vulgaris, Pernicious Anemia, Peyronie's Disease, Plasmacytoma/myeloma neuropathy, Poly-Dermatomyositis, Polyarteritis Nodosa, Polyendocrine Deficiency Syndrome, Polyendocrine Deficiency Syndrome Type 1, Polyendocrine Deficiency Syndrome Type 2, Polyglandular Autoimmune Syundrome Type I, Polyglandular Autoimmune Syndrome Type II, Polyglandular Autoimmune Syndrome Type III, Polymyositis, Primary Biliary Cirrhosis, Primary Glomerulonephritis, Primary Sclerosing Cholangitis, Psoriasis, Psoriatic Arthritis, Rasmussen's Encephalitis, Raynaud's Disease, Relapsing Polychondritis, Retrobulbar neuritis, Rheumatic Diseases, Rheumatoid Arthritis, Scleroderma, Sensory neuropathies (paraneoplastic), Sjogren's Syndrome, Stiff-Person Syndrome, Subacute Thyroiditis, Subacute autonomic neuropathy, Sydenham Chorea, Sympathetic Ophthalmitis, Systemic Lupus Erythematosus, Transverse myelitis, Type 1 Diabetes, Ulcerative Colitis, Vasculitis, Vitiligo, Wegener's Granulomatosis, acrocyanosis, anaphylactic reaction, autoimmune inner ear disease, bilateral sensorineural hearing loss, cold agglutinin haemolytic anemia, cold-induced immune haemolytic anemia, idiopathic endolymphatic hydrops, idiopathic progressive bilateral sensorineural hearing loss, immune-mediated inner ear disease, and mixed autoimmune hemolysis;
(b) the allergic reaction is chosen from systemic allergic reaction, allergic reaction to immunotherapy, anaphylactic reaction, atopic disease, contrast allergy, drug allergy, food allergy, hypersensitivity reaction, insect sting allergy, latex allergy, penicillin allergy, and radiocontrast medium allergy; optionally wherein the food allergy is selected from the group consisting of: peanut allergy and shellfish allergy;
(c) the transplant rejection is selected from the group consisting of: rejection following antigen transplantation; xenogenic transplantation and autologous transplantation of a tissue, an organs or cells into a subject.

5. The use of claim 1(a) or 1(b) or 3, wherein (a) the oxazaphosphorine drug is selected from the group consisting of: cyclophosphamide, ifosfamide, perfosfamide, trophosphamide, and a pharamaceutically acceptable salt, solvate, prodrug or metabolite thereof; optionally wherein the oxazaphosphorine drug is cyclophosphamide or a pharmaceutically acceptable salt or metabolite thereof; or (b) the lymphocytotoxic non-myeloablative amount of a oxazaphosphorine drug is:
(a) 50 mg/kg/day;
(b) administered to the subject for 4 days;
(c) 200 mg/kg administered over 4 consecutive days;
(d) 50mg/kg/day administered for 4 days; or
(e) cyclophosphamide administered in the amount of 50mg/kg for 4 days.

6. The use of claim 2 or 3, wherein the antimicrobial agent is chosen from the group consisting of Amdinocillin (Mecillinam), Amikacin, Amoxicillin, Ampicillin, Azithromycin, Aztreonam, Bacampicillin, Bacitracin, Carbenicillin indanyl sodium, Cefaclor, Cefadroxil, Cefamandole, Cefazolin, Cefdinir, Cefditoren, Cefepime, Cefixime, Cefinetazole, Cefonicid, Cefoperazone, Cefotaxime, Cefotetan, Cefoxitin, Cefpodoxime, Proxetil, Cefprozil, Ceftazidime, Ceftibuten, Ceftizoxime, Ceftriaxone, Cefuroxime, Cefuroxime axetil, Cephalexin, Cephalothin, Cephapirin, Cephradine, Chloramphenicol, Cinoxacin, Ciprofloxacin, Clarithromycin, Clindamycin, Cloxacillin, Colistimethate, Daptomycin, Demeclocycline, Dicloxacillin, Dirithromycin, Doxycycline, Enoxacin, Ertapenem, Erythromycin, Fosfomycin, Gatifloxacin, Gemifloxacin, Gentamicin, Grepafloxacin, Imipenem, Cilastatin, Kanamycin, Levofloxacin, Lincomycin, Linezolid, Lomefloxacin, Loracarbef, Mafenide, Meropenem, Methacycline, Methenamine mandelate, Methenamine hippurate, Methicillin, Metronidazole, Mezlocillin, Minocycline, Moxifloxacin, Mupirocin, Nafcillin, Nalidixic Acid, Neomycin, Netilmycin, Nitrofurantoin, Nitrofurazone, Norfloxacin, Novobiocin, Ofloxacin, Oxacillin, Oxytetracycline, Penicillin, Piperacillin, Polymyxin B, Rifamixin, Sparfloxacin, Spectinomycin, Streptomycin, Sulfadiazine, Sulfamethoxazole, Sulfisoxazole, Teicoplanin, Telithromycin, Tetracycline, Ticarcillin, Tobramycin, Trimethoprim, Trovafloxacin, Vancomycin, Amphotericin B, Amphotericin B Deoxycholate, Amphotericin B cholesteryl sulfate complex (ABCD), Amphotericin B lipid complex (ABLC), Amphotericin B liposomal, Caspofungin acetate, Clotrimazole, Fluconazole, Flucytosine, Griseofulvin, Itraconazole, Ketoconazole, Miconazole, Nystatin, Pentamidine, Terbinafine, Voriconazole, and pharmaceutically acceptable salts and derivatives thereof.

7. A kit for treating an immune disorder optionally chosen from an autoimmune disease, an allergic reaction and transplant rejection comprising:
(a) a plurality of doses of a non-myeloablative oxazaphosphorine drug; and
(b) instructions for treating the immune disorder using one or more doses of the oxazaphosphorine drug, wherein administration of the one or more doses is lymphocytotoxic;
optionally further comprising one or more of:
(i) a plurality of doses of granulocyte colony stimulating factor;
(ii) a plurality of doses of platelets; and
(iii) a plurality of doses of one or more antimicrobial agent;
optionally wherein the oxazaphosphorine drug is cyclophosphamide and wherein each of the plurality of doses is 50mg.

8. Use of a lymphocytotoxic non-myeloablative amount of a oxazaphosphorine drug for the preparation of a medicament for (i) treating a subject having multiple sclerosis, e.g., aggressive relapsing remitting multiple sclerosis, wherein (a) the subject failed to respond to conventional therapy, or (b) the subject has at least two gadolinium enhancing lesions; optionally wherein the oxazaphosphorine drug is cyclophosphamide; and optionally wherein the subject's immune system reconstitutes without stem cell transplantation and without administration of additional immunomodulatory agents; or (ii) treating a subject having multiple sclerosis, wherein treating the subject may further comprise:
(a) administering an effective amount of granulocyte colony stimulating factor to the subject;
(b) administering an effective amount of platelets to the subject; and
(c) administering an effective amount of at least one antimicrobial agent to the subject.

9. A kit for treating multiple sclerosis comprising:
(a) a plurality of doses of a non-myeloablative oxazaphosphorine drug; and
(b) instructions for treating multiple sclerosis using one or more doses of the oxazaphosphorine drug, wherein administration of the one or more doses is lymphocytotoxic;
optionally further comprising one or more of:
(i) a plurality of doses of granulocyte colony stimulating factor;
(ii) a plurality of doses of platelets; and
(iii) a plurality of doses of one or more antimicrobial agent (e.g., norfloxacin, fluconazole and valacyclovir);
optionally wherein the oxazapphosphorine drug is cyclophosphamide and wherein each of the plurality of doses is 50 mg.

10. A method of obtaining a cell population substantially free of cells capable of eliciting an adverse immune reaction in a subject, comprising:
(a) administering a lymphocytotoxic non-myeloablative amount of a oxazaphosphorine drug to the subject;
(b) administering an effective amount of granulocyte colony stimulating factor to the subject; and
(c) administering an effective amount of at least one antimicrobial agent to the subject, wherein step (a) is performed prior to steps (b)-(c) and wherein the method does not include both stem cell transplantation and administration of additional immunomodulatory agents;
optionally wherein effective amount of granulocyte colony stimulating factor is an amount, which results in a neutrophil count of at least 500/mm³, e.g., wherein the effective amount of granulocyte colony stimulating factor is 5 µg/kg/day; and optionally wherein the adverse immune reaction is chosen from an autoimmune disease, an allergic reaction and transplant rejection.

11. Use of a lymphocytotoxic non-myeloablative amount of a oxazaphosphorine drug for the preparation of a medicament for treating a subject having myasthenia gravis, wherein treating the subject may further comprise:
(a) administering an effective amount of granulocyte colony stimulating factor to the subject;
(b) administering an effective amount of platelets to the subject; and
(c) administering an effective amount of at least one antimicrobial agent to the subject;
optionally wherein the oxazaphosphorine drug is 50 mg/kg of cyclophosphamide for 4 consecutive days.

12. Use of a lymphocytotoxic non-myeloablative amount of a oxazaphosphorine drug for the preparation of a medicament for treating a subject having systemic lupus erythematosus, wherein treating the subject may further comprise:
(c) administering an effective amount of granulocyte colony stimulating factor to the subject;
(d) administering an effective amount of platelets to the subject; and
(e) administering an effective amount of at least one antimicrobial agent to the subject; optionally
wherein the oxazaphosphorine drug is 50 mg/kg of cyclophosphamide for 4 consecutive days.

13. Use of a lymphocytotoxic non-myeloablative amount of a oxazaphosphorine drug for the preparation of a medicament for treating a subject having autoimmune haemolytic anemia, wherein treating the subject may further comprise administering to the subject an effective amount of granulocyte colony stimulating factor to the subject; optionally wherein the oxazaphosphorine drug is 50 mg/kg of cyclophosphamide for 4 consecutive days.

14. Use of a lymphocytotoxic non-myeloablative amount of a oxazaphosphorine drug for the preparation of a medicament for treating a subject having autoimmune thrombocytopenia, wherein treating the subject may further comprise:
(a) administering to the subject an effective amount of granulocyte colony stimulating factor;
(b) administering to the subject an effective amount of platelets; and
(c) administering to the subject an effective amount of at least one antimicrobial agent,
and wherein there is no relapse for at least 4 years; optionally wherein the oxazaphosphorine drug is 50mg/kg of cyclophosphamide for 4 consecutive days.

15. Use of a lymphocytotoxic non-myeloablative amount of a oxazaphosphorine drug for the preparation of a medicament for treating a subject having pemphigus vulgaris, wherein treating the subject may further comprise:
(a) administering to the subject an effective amount of granulocyte colony stimulating factor;
(b) administering to the subject an effective amount of platelets; and
(c) administering to the subject an effective amount of at least one antimicrobial agent,
and wherein the subject's immune system reconstitutes without stem cell transplantation and without administration of additional immunomodulatory agents.
